# EUROPÄISCHE PATENTSCHRIFT

(11) **EP 1 953 130 B1**
(45) Veröffentlichungstag und Bekanntmachung des Hinweises auf die Patenterteilung: **29.06.2011**
(21) Anmeldenummer: 07001956.7
(22) Anmeldetag: 30.01.2007
(51) Int. Cl.: C07C 7/11, C07C 9/04, B01D 53/00

(54) **Verfahren und Anlage zur Behandlung von methan- und kohlendioxidhaltigen Rohgasen, insbesondere Biogas, zur Gewinnung von Methan**
Method and installation for raw gases containing processing methane and carbon dioxide, in particular biogas, for extracting methane
Procédé et installation de gaz brut contenant du dioxyde de carbone et de méthane, en particulier du biogaz, pour un gain de méthane

(43) Veröffentlichungstag der Anmeldung: 06.08.2008
(73) Patentinhaber: MT-Biomethan GmbH, 27404 Zeven (DE)
(72) Erfinder: Günther, Lothar, 82538 Geretsried (DE)
(74) Vertreter: Tragsdorf, Bodo

(56) Entgegenhaltungen:
- DE-B3-102005 051 952
- GB-A- 2 017 524

## Beschreibung

Die Erfindung bezieht sich auf ein Verfahren zur Behandlung von methan- und kohlendioxidhaltigen Rohgasen, Insbesondere Biogas, zur Gewinnung von Methan und eine zur Durchführung des Verfahrens geeignete Anlage.

Biogas entsteht durch anaerobe (sauerstofffreie) Vergärung von organischem Material und wird als regenerative Energiequelle genutzt. In Abhängigkeit von den Ausgangsstoffen, biomassehaltige Rohstoffe, Wirtschaftsdünger, wie Gülle und Mist, nachwachsende Rohstoffe, wird in Klärgas, Deponiegas und Biogas unterschieden.

Durch thermo-chemische Prozesse in der Industrie, wie die Vergasung, werden ebenfalls methanhaltige Gase gewonnen.

Auch das bei der Rohöldestillation anfallende Raffineriegas enthält Methan.

Vorgenannte Gase enthalten außerdem noch Kohlendioxid und Schwefelwasserstoff sowie geringe Restmengen anderer chemischer Stoffe.

Aus der DE 10 200 051 952 B3 ist ein Verfahren zur Herstellung von Methan und flüssigem Kohlendioxid aus Raffinerie- und/oder Biogas bekannt. Das Rohgas wird in einer Vorstufe gereinigt (Entfernung von Verunreinigungen wie NH₃, H₂SO₄, H₂S, SO₂ und COS) und anschließend einer Absorptionskolonne zugeführt, in der bei einem Druck von vorzugsweise 5 bis 30 bar unter Verwendung einer aminhaltigen Waschlösung im Rohgas enthaltenes Kohlendioxid in der Waschlösung gebunden wird. Das anfallende gereinigte Gas enthält ca. 98 Vol.-% Methan und kann einer direkten Weiterverwendung zugeführt werden. Die verunreinigte Waschlösung wird unter Druck und bei erhöhten Temperaturen (180 bis 230 °C) durch eine Desorptionskolonne regenerativ aufbereitet.

Der Nachteil dieser Verfahrensweise besteht darin, dass das Rohgas einer Vorreinigung unterzogen werden muss und das erhaltene Methan nur eine Reinheit von bis zu 98% besitzt und die Regeneration der Waschlösung einen hohen Energieaufwand erfordert. Außerdem ist die Durchführung der Aminwäsche des Rohgases unter erhöhtem Druck verfahrenstechnisch mit einem hohen apparativen Aufwand verbunden.

Der Erfindung liegt die Aufgabe zugrunde, ein Verfahren zur Behandlung von methan- und kohlendioxidhaltigen Rohgasen, Insbesondere Biogas, zur Gewinnung von Methan zu schaffen, das sich durch eine verbesserte Wirtschaftlichkeit auszeichnet und mit dem Methan in einer höheren Reinheit von über 99,5 Vol.-% erhalten wird und gleichzeitig der Energiebedarf für die Regeneration der Waschlösung deutlich gesenkt werden kann. Ferner soll eine zur Durchführung des Verfahrens geeignete Anlage geschaffen werden. Erfindungsgemäß wird die Aufgabe durch die im Anspruch 1 angegebenen Verfahrensmerkmale gelöst. Vorteilhafte Weiterbildungen der Verfahrensweise sind in den Ansprüchen 2 bis 9 angegeben. Eine zur Durchführung des Verfahrens geeignete Anlage ist Gegenstand des Anspruchs 10. Vorteilhafte Ausgestaltungen der Anlage sind in den Ansprüchen 11 bis 15 enthalten.

Gemäß der vorgeschlagenen Verfahrensweise wird das Rohgas, vorzugsweise Biogas, unter Normaldruck oder geringem Unterdruck (bis zu 50 mbar), vorzugsweise -20 (Unterdruck) bis 150 mbar, einer ein- oder mehrstufigen Wäsche mit einer aminhaltigen Waschlösung mit einer Aminkonzentration von mindesten 15% bei Normaltemperatur oder Temperaturen bis zu 100 °C, vorzugsweise 20 bis 60 °C, unterzogen. Aus dem in der Waschstufe entstehenden Reingasstrom aus Methan und Wasser wird durch nachfolgende Kühlung und Kondensation das Wasser abgetrennt und gegebenenfalls in den Waschkreislauf zurückgeführt.

Die Waschlösung wird im Kreislauf gefahren und einer speziellen regenerativen Aufarbeitung unterzogen, die zu einer besonders hohen Reinheit der Waschlösung führt.

Hierzu wird die aus der Waschstufe abgezogene, durch CO₂ und Schwefelverbindungen verunreinigte Waschlösung bis auf einen Reaktionsdruck von mindestens 4 bar, vorzugsweise 8 bis 12 bar, komprimiert und auf eine Temperatur oberhalb von 120 °C erhitzt. Nachfolgend wird diese in einer ersten Entspannungsstufe auf einen Druck, der 1 bis 3 bar unterhalb des Reaktionsdruckes liegt, entspannt. Unter Einhaltung einer Nachreaktionsdauer von 280 bis 1200 Sekunden und einer konstanten Reaktionstemperatur lässt sich der überwiegende Anteil an CO₂ und Schwefelverbindungen als Gasstrom aus der Waschlösung abtrennen. In dieser ersten Entspannungsstufe wird chemisch gebundenes CO₂ in der flüssigen Phase gelöst und entweicht. Die anfallende gereinigte Waschlösung wird auf Temperaturen unterhalb von 50 °C abgekühlt und in einer zweiten Entspannungsstufe auf Normaldruck entspannt. In dieser zweiten Entspannungsstufe können somit noch vorhandene Restmengen an lösbarem CO₂ und Schwefelverbindungen abgetrennt werden. In der zweiten Entspannungsstufe kommt es bei den vorliegenden Bedingungen (Temperatur und Druck) zu einer Rücklösung von noch physikalisch gebundenem CO₂, das aus der Waschlösung entweicht. Die Waschlösung besitzt nunmehr einen maximal möglichen Reinheitsgrad. Nachfolgend wird die vollständig gereinigte Waschlösung bis auf Normaltemperatur abgekühlt und in die Waschstufe zur Abtrennung von Methan aus Biogas zurückgeführt. Aufgrund der erforderlichen Kreislauffahrweise der Waschlösung kann das in der Waschstufe abgetrennte Methan nur mit einer Reinheit von mindestens 99,5% gewonnen werden, wenn die Waschlösung eine entsprechend hohe Reinheit besitzt.

Mit der vorgeschlagenen Verfahrenwelse ist es erstmals möglich, aus Biogas Methan in einer so hohen Reinheit zu gewinnen.

Bei einer mehrstufigen Abtrennung von Methan im Rahmen der Aminwäsche sollten in der ersten Stufe mehr als 50% der Insgesamt erforderlichen Menge an Waschlösung eingesetzt werden. Es ist zweckmäßig, die Waschlösungen der einzelnen Waschstufen vor der regenerativen Aufarbeitung zusammenzufassen.

Zur Aufheizung der Waschlösung auf die erforderliche Reaktionstemperatur ist es vorteilhaft, diese in zwei Stufen mittels indirekt beheizter Wärmetauscher durchzuführen. Dabei kann die aus der ersten Entspannungsstufe abgeschiedene heiße Waschlösung als Wärmeträger für mindestens einen der Wärmetauscher eingesetzt werden. In Abhängigkeit von der Temperatur der Waschlösung wird diese vorzugsweise als Wärmeträger für den ersten Wärmetauscher eingesetzt, da vorteilhafterweise diese dabei gleichzeitig auf die gewünschte Temperatur von mindestens 50 °C abgekühlt werden kann. Dies ist energetisch für die Verfahrensökonomie von großem Vorteil. In der ersten Entspannungsstufe können über 95% der in der Waschlösung gebundenen Anteile an CO₂ und Schwefelverbindungen abgeschieden werden.

Der in der ersten Entspannungsstufe abgetrennte, CO₂ und Sehwefelverbindungen enthaltende Gasstrom wird durch zwei in Reihe geschaltete Wärmetauscher geleitet und bis auf Normaltemperatur abgekühlt. Das im ersten Wärmetauscher als Wärmeträger eingesetzte Wasser wird dabei auf eine deutlich höhere Temperatur erwärmt und das so erhaltene warme Wasser kann effektiv für andere Zwecke genutzt werden. Auf diese Weise werden ca. 60% der unmittelbar vor der ersten Entspannungsstufe zugeführten Wärmeenergie wieder zurückgewonnen.

Nach erfolgter Abkühlung des Gasstromes wird aus diesem in einem nachgeschalteten Abscheider Wasser abgeschieden, das wieder in den Kreislauf der Waschlösung zurückgeführt wird, durch Einleitung in den Abscheider der zweiten Entspannungsstufe. Der wasserfreie Gasstrom (CO₂ und Schwefelverbindungen) wird druckgeregelt, z.B. an die Umgebung, abgeführt. Bei einem zu hohen Schwefelgehalt des Gasstromes wird dieser in einer nachgeschalteten Entsehwefelungsanlage entschwefelt.

Die Reaktionszeiten in der ersten Entspannungsstufe können durch eine Ultraschallbehandlung der Waschlösung weiter reduziert werden. Die Verweilzeit in dieser Stufe kann bei gleichem Abscheideergebnis um bis zu ca. 30% verringert werden.

Eine zur Durchführung des Verfahrens geeignete Anlage besteht aus einer ein- oder mehrstufige Waschkolonne zur Abtrennung von Methan aus dem zu behandelnden Rohgas. Die Waschkolonne steht mit einer Kreislaufleitung für die Waschlösung in Verbindung. In diese sind in Strömungsrichtung der Waschlösung eine erste Kreislaufpumpe, nachfolgend mindestens ein Wärmetauscher zur Erwärmung der Waschlösung und anschließend eine erste und eine zweite Entspannungsvorrlchtung eingebunden. In der von der zweiten Entspannungsvorrlchtung abführenden Leitung für die gereinigte Waschlösung ist eine zweite Kreislaufpumpe eingebunden. Außerdem ist in die Kreislaufleitung nach der zweiten Entspannungsvorrichtung ein Wärmetauscher zur Kühlung der Waschlösung auf Normaltemperatur eingebunden. Nach dem Verlassen dieses Wärmetauschers gelangt die hochreine Waschlösung wieder in die Waschkolonne.

Es hat sich als vorteilhaft herausgestellt, wenn die Waschlösung vor der Einleitung in die erste Entspannungsstufe durch zwei in Reihe geschaltete Wärmetauscher auf die erforderliche Temperatur erhitzt wird. Die beiden Entspannungsvorrichtungen sind mit Gasstromleitungen zur Abführung von CO₂ und Schwefelverbindungen verbunden. Die Gasstromleitung der ersten Entspannungsvorrichtung ist mit einem Abscheider verbunden, wobei zwischen dieser und dem Abscheider zwei in Reihe geschaltete Wärmetauscher zur Kühlung des Gasstromes zwischengeschaitet sind.

Der Abscheider ist mit einer Leitung zur Abführung des Gasstromes und einer Leitung zur Weiterleitung des abgeschiedenen Kondenswassers in die zweite Entspannung vorrichtung verbunden.

In die von der ersten Entspannungsvörrichtung abzweigende Leitung für die Waschlösung ist mindestens einer der vorgeschalteten Wärmetauscher eingebunden, wobei die Waschlösung diesen als Wärmeträger durchströmt und dabei gleichzeitig abgekühlt wird, Erforderlichenfalls können die Gastromleitungen, die schwefelhaltige Gase abführen, an eine Entschwefelungsanlage angeschlossen werden.

Die vorgeschlagene Anlage kann für einen breiten Leistungsbereich genutzt werden und zeichnet sich durch einen energetisch hohen Wirkungsgrad aus.

Die Erfindung soll nachstehend an einem Ausführungsbeispiel erläutert werden. In der zugehörigen Zeichnung ist das Funktionsschema einer Anlage zur Behandlung von Biogas gezeigt.

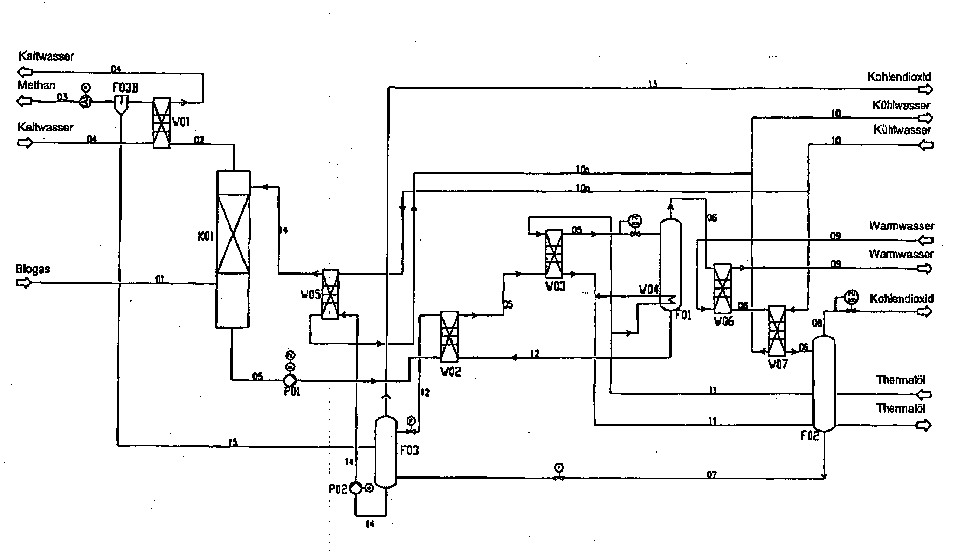

Die gezeigte Anlage besteht aus einer ein- oder mehrstufigen Waschkolonne K01, wobei die in der Zeichnung dargestellte Waschkolonne einstufig ausgebildet ist. Die Waschkolonne K01 ist mit einer Leitung 01 zur Zuführung von Biogas verbunden. Im oberen Teil der Waschkolonne K01 ist eine Leitung 02 zur Abführung von in der Waschkolonne K01 abgetrenntem wasserhaltigem Methan vorgesehen. In einem nachgeschalteten Wärmetauscher W01 kondensiert im Methan enthaltenes Wasser. Über eine Leitung 03 wird das vom Wasser befreite Methan abgezogen. In die Leitung 03 ist zur Entfeuchtung des Methans ein Wasserabscheider F03B eingebunden. Das abgeschiedene Wasser wird Ober eine Leitung 15 in die zweite Entspannungsvorrichtung F03 geleitet.

Der Wärmetauscher W01 wird über eine Kreislaufleitung 04 mit Kaltwasser gespeist, das in einer nicht näher gezeigten Kühleinrichtung gekühlt wird.

Am Boden der Waschkolonne K01 wird Ober eine Leitung 05 Waschlösung, in der CO₂ und Schwefelverbindungen gebunden sind, mittels einer Kreislaufpumpe P01 zu einem ersten Wärmetauscher W02 gepumpt, von diesem weiter zu einem zweiten Wärmetauscher W03 und von diesem zu einer ersten Entspannungsvorrichtung F01, in der aus der Waschlösung der überwiegende Anteil an CO₂ und Schwefelverbindungen durch eine Flashentspannung abgetrennt werden. Zur Konstanthaltung der erforderlichen Reaktionstemperatur ist in die Flashentspannungsvorrichtung F01 ein Wärmetauscher W04 eingebunden.

CO₂ und Schwefelverbindungen werden am Kopf der Flashentspannungsvorrichtung F01 über die Leitung 06 abgezogen und zur weiteren Abkühlung durch einen dritten und vierten Wärmetauscher W06 und W07 geleitet. In einem nachgeschalteten Abscheider F02 wird kondensiertes Wasser über die Leitung 07 abgefühlt und über die Leitung 08 CO₂ und Schwefelverbindungen druckgeregelt an die Umgebung abgelassen. Der Wärmetauscher W06 wird über eine Kreislaufleitung 09 mit Warmwasser gespeist und der Wärmetauscher 07 Ober eine Kreislaufieltung 10 mit Kaltwasser. Dem Wärmetauscher W03 wird über eine Kreislaufleitung 11 Thermalöl zu- und abgeführt. Die während der Flashentspannung freiwerdende Kondensationswärme wird zur Erhitzung des Wärmetauscher W06 genutzt. Ca. 60% der Im Wärmetauscher W03 zugeführten Wärmeenergie werden somit wieder zurück gewonnen, als Heißwasser, das einem gesonderten Verwendungszweck zugeführt werden kann.

Über eine Leitung 12 wird die heiße, gereinigte Waschlösung aus der Flashentspannungsvorrichtung F01 als Wärmeträger zum Wärmetauscher W02 geleitet und gelangt von diesem aus druckgeregelt In eine zweite Entspannungsvorrichtung, den Abscheider F03. Das Im Abscheider F02 anfallende Kondenswasser wird über die Leitung 07 druckgeregelt dem Abscheider F03 zugeführt. In diesem werden noch in der Waschlösung und/oder Kondenswasser enthaltenes CO₂ und Schwefelverbindungen abgetrennt und über die Leitung 13 an die Umgebung abgelassen. Die Im Abscheider F03 anfallende Waschlösung wird über die Leitung 14 mittels der Kreislaufpumpe P02 zum Wärmetauscher W05 gepumpt und nach erfolgter Abkühlung In die Waschkolonne K01 wieder zurückgeführt. Zur Kühlung des Wärmetauschers W05 ist dieser über die Leitungen 10a in die Kreislaufleitung 10 eingebunden. Die beschriebene Anlage ermöglicht eine besonders wirtschaftliche und energiesparende Fahrweise zur Behandlung von Biogas.

Nachfolgend wird die Verfahrensweise der Anlage erläutert.

Im Rahmen einer Biogasproduktion entsteht Biogas, welches vorentschwefelt wurde und aus dem auch andere störende Komponenten ohne Zuführung von Sauerstoff oder Luft entfernt wurden. Ein herkömmliches Biogas besitzt beispielsweise folgende Zusammensetzung:

| | |
|---|---|
| Methan | 52 Vol.-% |
| Kohlendioxid | 44 Vol.-% |
| Wasser | 3 Vol.-% |
| Wasserstoff | 0,1 Vol.-% |
| Schwefelverbindungen | 0,2 Vot.-% als H₂S und COS (organische Schwefelver |
| bindungen) oder Spuren im Bereich unter 2 ppm an Schwefel-Verbindungen, Ammoniak. | |

Das zu behandelnde Biogas (500 Nm³/h; N=Nortnzustand) wird unter den gegebenen Bedingungen bei einer Temperatur von 20 bis 60 °C direkt (ohne zusätzliche Vorreinigung) der Waschkolonne K01 zugeführt. In dieser wird unter Normaldruck oder geringem Vakuum (-10 bis 150 mbar) der Waschprozess zur Entfernung von CO₂, H₂S und COS aus dem Biogas durchgeführt. Dieser erfolgt mit einer Waschlösung, die mindestens eine Aminkomponente, vorzugsweise Diethanolamin, mit einer Konzentration von 15 bis 50% enthält. Die Einsatzmenge an Waschlösung ist abhängig von der Aminkonzentration, der Wasseranteil sollte mindesten 20% betragen. Bei einem Einsatz einer Waschlösung mit einer Aminkonzentration von 20% werden zur Reinigung von 500 Nm³/h Biogas ca. 15 m³/h an Waschlösung benötlgt. Bei einer Waschlösung mit einem Amingehalt von 30% beträgt die erforderliche Einsatzmenge 9 m³/h. Die im Biogas enthaltenen Schwefelverbindungen und CO₂ werden beim Inkontaktbringen mit der Waschlösung vollständig in dieser gebunden. Das aus der Waschkolonne K01 über die Leitung 02 austretende gereinigte Biogas besteht aus Methan und Wasser, mit einem sehr geringen Restanteil (0,2% oder kleiner) an CO₂. In einer nachgeschalteten Kühlstufe (Wärmetauscher W01) kondensiert im Methan enthaltenes Wasser.

Das kondensierte Wasser wird zum Waschkreislauf zurückgeführt und damit der gleichbleibende Wasseranteil in der Waschlösung eingestellt. Das noch feuchte Methan wird in einem Wasserabscheider F03B entfeuchtet und das abgetrennte Wasser über die Leitung 15 In die zweite Entspannungsvorrichtung F03 zurückgeführt.

Das abgetrennte Methan hat eine Reinheit von über 99,5 Vol.-% und kann durch zusätzliche Trocknung als praktisch 100% reines Methan aufgearbeitet werden.

Die am Boden der Waschkolonne K01 anfallende Waschlösung (ca. 15 m³/h, Aminkonzentration 20%) wird auf einen Druck von ca. 8,5 bar komprimiert, mittels der Kreislaufpumpe P01 In einen ersten Wärmetauscher W02 geleitet und In diesem durch indirekten Wärmetausch auf eine Temperatur von ca. 145 °C erwärmt. Als Wärmetauschermedium wird gereinigte Waschlösung eingesetzt, die nach der Flashentspannung F01 anfällt. Die aus dem Wärmetauscher W02 abgeführte Waschlösung wird anschließend In einem zweiten Wärmetauscher W03 auf eine Temperatur von ca. 165 °C erhitzt. Der Wärmetauscher W03 wird von einem externen Wärmeträger, wie z.B. Thermalöl, durchströmt.

Die Waschlösung wird anschließend in einer ersten Entspannungsstufe, der Flashentspannungsvorrichtung F01, auf einen Druck von ca. 6,5 bar entspannt. Dabei reduziert sich der Anteil von chemisch gebundenem Kohlendioxid und Schwefelverbindungen im Waschwasser auf ca. 1/10 während einer Nachreaktionszeit von 480 und einer Reaktionstemperatur von ca. 165 °C. Unter diesen Bedingungen reduziert sich der Anteil von chemisch gebundenem CO₂ und Schwefel von 45 g/l auf 5 g/l.

Unter anderen verfahrenstechnischen Bedingungen kann die Nachreaktionszeit auch 280 s bis 1200 s betragen, wobei aus verfahrensökonomischer Sicht kurze Nachreaktionszeiten von Vorteil sind. Die Konstanthaltung der Reaktionstemperatur erfolgt durch einen in die Flashentspannungsvorrichtung F01 eingebundenen Wärmetauscher W04. Die Flashentspannung sollte vorzugsweise bei einer Flashtemperatur erfolgen, die 1 bis 10 °C unterhalb der Verdampfungstemperatur von Wasser liegt, bei einer Entspannung von ca. 8,5 bar auf ca. 6,5 bar und einer Temperatur von ca. 165 °C.

Die gereinigte Waschlösung, die nur noch geringe Mengen an CO₂ und Schwefelverbindungen enthält und eine Temperatur von ca. 165 °C hat, wird über die Leitung 12 dem Wärmetauscher W02 als Wärmeträger zugeführt und dabei auf eine Temperatur von 34 °C abgekühlt. Nachfolgend wird die Waschlösung in einer zweiten Entspannungsstufe (Abscheider F03) auf Normaldruck entspannt. Dabei entweichen am Kopf des Abscheiders die noch in der Waschlösung enthaltenen Restmengen an CO₂ und Schwefelverbindungen, die in die Umgebung abgelassen werden können. Mittels der Kreislaufpumpe P02 wird die vollständig gereinigte Waschlösung dem Wärmetauscher W05 zugeführt und in diesem auf Normaltemperatur abgekühlt und anschließend In die Waschkolonne K01 zurückgeführt.

In der zweiten Entspannungsstufe wird eine Rücklösung von noch physikalisch gebundenem CO₂ bewirkt, das über die Leitung 13 entweicht. Durch die zweite Entspannungsstufe F03 wird ein maximal möglicher Reinheitsgrad der Waschlösung erzielt. Da die Waschlösung im Kreislauf gefahren wird und nach der Reinigung wieder In die Waschstufe K01 gelangt, beeinflusst deren Reinheitsgrad die Reinheit des In der Waschstufe abgetrennten Methans. Nur durch einen hohen Reinheitsgrad der zurückgeführten Waschlösung lässt sich aus Biogas ein Methan mit einer Reinheit von mindestens 99,5% erzielen.

In der hochreinen Waschlösung sind noch ca. 10% an CO₂ chemisch gebunden, die sich nur schwer abtrennen lassen, mit einem wirtschaftlich nicht vertretbaren Aufwand.

Das am Kopf der Flashentspannungseinrichtung F01 austretende Gasgemisch aus Kohlendioxid, Wasser und Schwefelverbindungen wird Im Wärmetauscher W06 auf eine Temperatur von 60 °C und Im nachfolgenden Wärmetauscher W07 bis auf Normaltemperatur (ca. 25 - °C) abgekühlt. Anschließend wird Im Abscheider F02 kondensiertes Wasser abgeschieden. Dieses wird in dosierter Menge Ober die Leitung 07 In den Abscheider F03 geleitet und vermischt sich in diesem mit der vollständig gereinigten Waschlösung. Das am Kopf des Abscheiders F02 abströmende Gasgemisch (CO₂ und Schwefelverbindungen) wird druckgeregelt in die Umgebung abgelassen.

Sind im abgetrennten Gasgemisch unzulässig hohe Konzentrationen an Schwefelverbindungen enthalten, so wird noch eine nachgeschaltete Schwefelentfernung, z.B. mittels Blofilter, Adsorption oder als physikalischich/chemische oder biologische Wäsche durchgeführt. Die Reaktionsdauer der Im Abscheider F01 stattfindenden chemischen Zersetzung der Waschlösung kann durch eine zusätzliche Ultraschallbehandlung deutlich verkürzt werden, beispielsweise um 70%, bei gleichen Temperatur- und Druckbedingungen.

Wie bereits erwähnt, kann die Waschstufe K01 zur Abtrennung des Methans mehrstufig, vorzugsweise zweistufig, ausgebildet sein. Die hierzu erforderlichen Waschkolonnen sind In Reihe geschaltet. In der ersten Waschstufe sollte die Einsatzmenge der Waschlösung, bezogen auf die insgesamt erforderliche Menge, mehr als 50% betragen. Die in beiden Waschstufen anfallenden Waschlösungen werden zusammengeführt und in der vorbeschrlebenen Weise regenerativ aufgearbeitet.

Ausgehend von einer Einsatzmenge an Biogas von 500 Nm³/h und einer Einsatzmenge an Waschlösung (Aminkonzentration 20%) von 15 m³/h, werden unter vorgenannten Bedingungen 260 m³/h an Methan mit einer Reinheit von mindestens 99,5% erhalten. Die eingesetzte Menge an Waschlösung kann mittels der vorgeschlagenen regenerativen Aufarbeitung in maximal möglicher Reinheit zu nahezu 100% wieder zurückgewonnen und im Kreislauf gefahren werden.

## Patentansprüche

1. Verfahren zur Behandlung von methan- und kohlendioxidhaftigen Rohgasen zur Gewinnung von Methan, wobei das Rohgas zur Abtrennung von Methan mit einer aminhaltigen Waschlösung behandelt wird, im Rohgas enthaltenes CO₂ In der Waschlösung gebunden und die verunreinigte Waschlösung regenerativ aufgearbeitet wird, **dadurch gekennzeichnet, dass**
a) das Rohgas unter Normaldruck oder geringem Unterdruck (bis zu 50 mbar) einer ein- oder mehrstufigen Wäsche (K01) mit einer aminhaltigen Waschlösung mit einer Aminkonzentration von mindesten 15% bei Normaltemperatur oder Temperaturen bis zu 100 °C unterzogen wird unter Bildung eines aus Methan und Wasser bestehenden Reingasstromes, aus dem durch nachfolgende Kühlung und Kondensation das Wasser abgetrennt und gegebenenfalls in den Waschkreislauf zurückgeführt wird;
b) die nach a) erhaltene CO₂ und Schwefelverbindungen enthaltende Waschlösung bis auf einen Reaktionsdruck von mindestens 4, vorzugsweise 8 bis 12 bar, komprimiert und auf eine Temperatur oberhalb von 120 °C erhitzt wird und in einer ersten Entspannungsstufe (F01) auf einen Druck, der 1 bis 3 bar unterhalb des Reaktionsdruckes liegt, entspannt wird, unter Einhaltung einer Nachreaktionsdauer von 280 bis 1200 Sekunden und einer konstanten Reaktionstemperatur, wobei der überwiegende Anteil an CO₂ und Schwefelverbindungen aus der Waschlösung abgetrennt und als Gasstrom abgezogen wird;
c) die gereinigte Waschlösung auf Temperaturen unterhalb von 50 °C abgekühlt und In einer zweiten Entspannungsstufe (F03) auf Normaldruck entspannt wird, wobei noch vorhandene Restmengen an lösbarem CO₂ und Schwefelverbindungen abgetrennt werden und die vollständig gereinigte Waschlösung bis auf Normaltemperatur abgekühlt und in die Waschstufe (K01) zurückgeführt wird.

2. Verfahren nach Anspruch 1, **dadurch gekennzeichnet, dass** bei einer mehrstufigen Wäsche in der ersten Waschstufe mehr als 50% der Insgesamt erforderlichen Menge an Waschlösung eingesetzt werden und die Waschlösungen der einzelnen Waschstufen zusammengefasst und regenerativ aufbereitet werden.

3. Verfahren nach einem der Ansprüche 1 oder 2, **dadurch gekennzeichnet, dass** die nach der Wäsche erhaltene Waschlösung in zwei Stufen mittels Indirekt beheizter Wärmetauscher (W02, W03) auf die erforderliche Reaktionstemperatur erwärmt wird.

4. Verfahren nach einem der Ansprüche 1 bis 3, **dadurch gekennzeichnet, dass** der in der ersten Entspannungsstufe (F01) abgetrennte, CO₂ und Schwefelverbindungen enthaltende Gasstrom durch zwei in Reihe geschaltete Wärmetauscher (W06, W07) geleitet und bis auf Normaltemperatur abgekühlt wird und nachfolgend einem Abscheider (F02) zugeführt wird, in dem aus dem Gasstrom kondensiertes Wasser abgeschieden und der wasserfreie Gasstrom (CO₂ und Schwefelverbindungen) druckgeregelt abgeführt wird.

5. Verfahren nach einem der Ansprüche 1 bis 4, **dadurch gekennzeichnet, dass** die aus der ersten Entspannungsstufe (F01) abgeschiedene heiße Waschlösung als Wärmeträger für mindestens einen der Wärmetauscher (W02) eingesetzt und dabei auf eine Temperatur von mindestens 50 °C abgekühlt wird.

6. Verfahren nach einem der Ansprüche 1 bis 5, **dadurch gekennzeichnet, dass** in der Verfahrensstufe a) das Rohgas auf eine Temperatur von 20 bis 60 °C erwärmt wird und ein Druck im Bereich von -20 bis 150 mbar eingestellt wird.

7. Verfahren nach einem der Ansprüche 1 bis 6, **dadurch gekennzeichnet, dass** in der ersten Entspannungsstufe (F01) über 95°% der in der Waschlösung gebundenen Anteile an CO₂ und Schwefelverbindungen abgeschieden werden.

8. Verfahren nach einem der Ansprüche 1 bis 7, **dadurch gekennzeichnet, dass** mindestens der aus der ersten Entspannungsstufe (F01) abgetrennte Gasstrom in einer nachgeschalteten Entschwefelungsanlage entschwefelt wird.

9. Verfahren nach einem der Ansprüche 1 bis 8, **dadurch gekennzeichnet, dass** während der ersten Entspannungsphase (F01) die Waschlösung einer Ultraschallbehandlung ausgesetzt wird.

10. Anlage zur Durchführung des Verfahrens nach mindestens einem der vorgenannten Ansprüche, **dadurch gekennzeichnet, dass** diese aus einer ein- oder mehrstufigen Waschkolonne (K01) zur Abtrennung von Methan aus dem zu behandelnden Rohgas besteht, die mit einer Kreislaufleitung (05, 12, 14) für die Waschlösung in Verbindung steht, in die eine erste Kreislaufpumpe (P01), in Strömungsrichtung nach der Kreislaufpumpe (P01) mindestens ein Wärmetauscher (W02 oder W03) zur Erwärmung der Waschlösung und nachfolgend eine erste (F01) und zweite Entspannungsvorrichtung (F03) eingebunden sind, wobei in der von der zweiten Entspannungsvorrtchtung (F03) abführenden Leitung (14) für die gereinigte Waschlösung eine zweite Kreislaufpumpe (P02) und ein Wärmetauscher (W05) zur Kühlung der Waschlösung auf Normaltemperatur eingebunden sind.

11. Anlage nach Anspruch 10, **dadurch gekennzeichnet, dass** zur stufenweise Erwärmung der Waschlösung vor der ersten Entspannungsvorrichtung (F01) zwei in Reihe geschaltete Wärmetauscher (W02, W03) angeordnet sind.

12. Anlage nach einem der Ansprüche 10 oder 11, **dadurch gekennzeichnet, dass** die Entspannungsvorrichtungen (F01, F03) mit Leitungen (06, 13) zur Abführung eines Gasstromes (CO₂ und Schwefelverbindungen) verbunden sind.

13. Anlage nach einem der Ansprüche 10 bis 12, **dadurch gekennzeichnet, dass** die Gasstromleitung (06) der ersten Entspannungsvorrichtung (F01) mit einem Abscheider (F02) verbunden ist, wobei zwischen der Entspannungsvorrichtung (F01) und dem Abscheider (F02) zwei in Reihe geschaltete Wärmetauscher (W06, W07) zur Kühlung des Gasstromes eingebunden sind, der Abscheider (F02) mit einer Leitung (08) zur Abführung des Gasstromes und einer Leitung (07) zur Weiterleitung des abgeschiedenen Kondenswassers in die zweite Entspannungsvorrichtung (F03) verbunden ist.

14. Anlage nach einem der Ansprüche 10 bis 13, **dadurch gekennzeichnet, dass** in die von der ersten Entspannungsvorrichtung (F01) abzweigende Leitung (12) für die Waschlösung mindestens einer der vorgeschalteten Wärmetauscher (W02) eingebunden ist, wobei die Waschlösung diesen als Wärmeträger durchströmt.

15. Anlage nach einem der Ansprüche 10 bis 14, **dadurch gekennzeichnet, dass** mindestens die Gasstromleitung (08), die vom nachgeschalteten Abscheider (F02) abzweigt, an eine Entschwefelungsanlage angeschlossen ist.

## Claims

1. Method of treating crude gases containing methane and carbon dioxide in order to produce methane in which the crude gas is treated with an amine-containing scrubbing solution to remove the methane, the CO₂ contained in the crude gas is bound in the scrubbing solution and the contaminated scrubbing solution is regeneratively processed, **characterised in that**
a) the crude gas is subjected under standard pressure or a slight negative pressure (up to 50 mbar) to a single or multi-stage scrubbing process (K01) with an amine-containing scrubbing solution at an amine concentration of at least 15% at standard temperature or temperatures up to 100°C with the formation of a pure gas stream consisting of methane and water from which the water is removed and if necessary returned to the scrubbing circuit by downstream cooling and condensation;
b) the scrubbing solution containing CO₂ and sulphur compounds obtained as in a), is compressed up to a reaction pressure of at least 4, preferably 8 to 12 bar, and heated to a temperature above 120°C and expanded in a initial expansion stage (F01) to a pressure which is 1 to 3 bar below the reaction pressure, while maintaining a secondary reaction time of 280 to 1,200 seconds and a constant reaction temperature, with the major proportion of the CO₂ and sulphur compounds removed from the scrubbing solution and drawn off as a gas stream
c) the purified scrubbing solution is cooled to temperatures below 50°C and expanded in a second expansion stage (F03) to standard pressure, with residual amounts of soluble CO₂ and sulphur compounds still present removed and the completely purified scrubbing solution cooled to standard temperature and returned to the scrubbing stage (K01).

2. Method according to claim 1, **characterised in that** in a multi-stage scrubbing process more than 50% of the total amount of scrubbing solution required is used in the initial scrubbing stage and the scrubbing solutions of the individual scrubbing stages are combined and regeneratively processed.

3. Method according to one of the claims 1 or 2, **characterised in that** the scrubbing solution obtained after washing is heated to the required reaction temperature in two stages by indirectly heated heat exchangers (W02, W03).

4. Method according to one of the claims 1 to 3, **characterised in that** the gas stream containing CO₂ and sulphur compounds separated in the initial expansion stage (F01) is conducted through two heat exchangers connected in series (W06, W07) and cooled to standard temperature and conveyed to a downstream separator (F02) in which water condensed from the gas stream water is removed and the water-free gas stream (CO₂ and sulphur compounds) is removed under controlled pressure.

5. Method according to one of the claims 1 to 4, **characterised in that** the hot scrubbing solution removed from the initial expansion stage (F01) is used as a heat carrier for at least one of the heat exchangers (W02) and is cooled in the process to a temperature of at least 50°C.

6. Method according to one of the claims 1 to 5, **characterised in that** the crude gas is heated to a temperature of 20 to 60°C in the process stage a) and adjusted to a pressure ranging from -20 to 150 mbar.

7. Method according to one of the claims 1 to 6, **characterised in that** over 95% of the proportions of CO₂ and sulphur compounds bound in the scrubbing solution are removed in the initial expansion stage (F01).

8. Method according to one of the claims 1 to 7, **characterised in that** at least the gas stream removed from the initial expansion stage (F01) is desulphurised in a downstream desulphurisation plant.

9. Method according to one of the claims 1 to 8, **characterised in that** the scrubbing solution is subjected to an ultrasonic treatment during the initial expansion phase (F01).

10. Plant for carrying out the method according to at least one of the aforementioned claims, **characterised in that** the plant comprises of a single or multi-stage scrubbing column (K01) for removing methane from the crude gas to be treated, said scrubbing column is being connected to a circulation line (05, 12, 14) for the scrubbing solution, in which are integrated a first circulation pump (P01), at least one heat exchanger (W02 or W03) in the direction of flow downstream of the circulation pump (P01) for heating the scrubbing solution and further downstream a first (F01) and a second expansion device (F03), in which a second circulation pump (P02) and a heat exchanger (W05) for cooling the scrubbing solution to standard temperature are integrated in the line (14) for the purified scrubbing solution branching off from said second expansion device (F03).

11. Plant according to claim 10, **characterised in that** two heat exchangers (W02, W03) connected in series are arranged upstream of the first expansion device (F01) for the gradual heating of the scrubbing solution.

12. Plant according to one of the claims 10 or 11, **characterised in that** the expansion devices (F01, F03) are connected to lines (06, 13) for removing a gas stream (CO₂ and sulphur compounds).

13. Plant according to one of the claims 10 to 12, **characterised in that** the gas flow line (06) of the first expansion device (F01) is connected to a separator (F02), with two heat exchangers (W06, W07) connected in series for cooling the gas stream integrated between the expansion device (F01), and with the separator (F02), and the separator (F02) connected to a line (08) for removing the gas stream and to a line (07) for conveying the separated condensation water into the second expansion device (F03).

14. Plant according to one of the claims 10 to 13, **characterised in that** at least one of the upstream heat exchangers (W02) is integrated into the line (12) for the scrubbing solution branching off from the first expansion device (F01), with the scrubbing solution flowing through said heat exchanger as a heat carrier.

15. Plant according to one of the claims 10 to 14, **characterised in that** at least the gas flow line (08) branching off from the downstream separator (F02) is connected to a desulphurisation plant.

## Revendications

1. Procédé pour le traitement de gaz bruts contenant du méthane et du dioxyde de carbone pour la récupération du méthane, le gaz brut étant traité, pour la séparation du méthane, à l'aide d'une solution d'amine lavante, le CO₂ contenu dans le gaz brut étant lié dans la solution lavante et la solution lavante impure étant traitée par régénération, **caractérisé en ce que**
(a) le gaz brut est soumis, à pression normale ou faible dépression (jusqu'à 60 mbar), à un lavage (K01) en une seule étape ou en plusieurs étapes à l'aide d'une solution d'amine lavante d'une concentration en amine d'au moins 15% à température normale ou à des températures allant jusqu'à 100°C, en formant un courant de gaz pur composé de méthane et d'eau duquel est séparée l'eau par refroidissement et condensation consécutifs et est ramenée le cas échéant dans le circuit de lavage ;
(b) la solution lavante contenant le CO₂ et les composés de soufre, obtenue d'après a), est comprimée jusqu'à obtention d'une pression réactionnelle d'au moins 4 bar, de préférence entre 8 et 12 bar, et est portée à une température supérieure à 120°C et est détendue au cours d'une première étape de détente (F01) jusqu'à obtention d'une pression inférieure de 1 à 3 bar à la pression réactionnelle, en maintenant une durée postréactionnelle comprise entre 280 et 1 200 secondes et une température réactionnelle constante, la plus grande part de CO₂ et de composés de soufre étant séparée de la solution lavante et évacuée sous forme de courant de gaz ;
(c) la solution lavante purifiée est refroidie jusqu'à obtention de températures inférieures à 50°C et détendue au cours d'une seconde étape de détente (F03) jusqu'à obtention d'une pression normale, des quantités restantes encore présentes de CO₂ soluble et de composés de soufre étant séparées et la solution lavante entièrement purifiée étant refroidie jusqu'à obtention d'une température normale et est ramenée à l'étape de lavage (K01).

2. Procédé selon la revendication 1, **caractérisé en ce que**, lors d'un lavage en plusieurs étapes, plus de 50% de la quantité totale nécessaire de solution lavante sont utilisés au cours de la première étape de lavage et les solutions lavantes de chacune des étapes de lavage sont rassemblées et traitées par régénération.

3. Procédé selon l'une quelconque des revendications 1 ou 2. **caractérisé en ce que** la solution lavante obtenue après le lavage est portée à la température ambiante nécessaire en deux étapes au moyen d'échangeurs de chaleur (W02, W03) chauffés indirectement.

4. Procédé selon l'une quelconque des revendications 1 à 3, **caractérisé en ce que** le courant de gaz contenant le CO₂ et les composés de soufre, séparé au cours de la première étape de détente (F01), traverse deux échangeurs de chaleur (W06, W07) montés en série et est refroidi jusqu'à obtention d'une température normale et est amené ensuite à un séparateur (F02) dans lequel l'eau condensée est séparée du courant de gaz et le courant de gaz exempt d'eau (CO₂ et composés de soufre) est éliminé en réglant la pression.

5. Procédé selon l'une quelconque des revendications 1 à 4, **caractérisé en ce que** la solution lavante chaude, séparée au cours de la première étape de détente (F01), est utilisée sous forme d'agent caloporteur pour au moins l'un des échangeurs de chaleur (W02) en étant refroidie jusqu'à obtention d'une température d'au moins 50°C.

6. Procédé selon l'une quelconque des revendications 1 à 5, **caractérisé en ce qu'**au cours de l'étape de procédé a), le gaz brut est porté à une température comprise entre 20 et 60°C et une pression comprise dans une plage allant de 20 à 150 mbar est réglée.

7. Procédé selon l'une quelconque des revendications 1 à 6, **caractérisé en ce qu'**au cours de la première étape de détente (F01), on a une séparation de plus de 95% des parts de CO₂ et de composés de soufre liées dans la solution lavante.

8. Procédé selon l'une quelconque des revendications 1 à 7, **caractérisé en ce qu'**au moins le courant de gaz séparé au cours de la première étape de détente (F01) est désulfuré dans un dispositif de désulfuration placé à la suite.

9. Procédé selon l'une quelconque des revendications 1 à 8, **caractérisé en ce qu'**au cours de la première étape de détente (F01), la solution lavante est soumise à un traitement par ultrasons.

10. Installation pour la mise en oeuvre du procédé selon au moins l'une quelconque des revendications précitées, **caractérisée en ce que** celle-ci se compose d'une colonne de lavage (K01) en une seule ou plusieurs étapes pour l'extraction du méthane hors du gaz brut à traiter qui est reliée à une conduite de circuit (05, 12, 14) pour la solution lavante dans laquelle sont intégrés une première pompe de circuit (P01), dans le sens du courant après la pompe de circuit (P01) au moins un échangeur de chaleur (W02 ou W03) pour réchauffer la solution lavante, et à la suite de celui-ci un premier (F01) et un second dispositif de détente (F03), une seconde pompe de circuit (P02) et un échangeur de chaleur (W05) pour le refroidissement de la solution lavante à température normale étant intégrés dans la conduite (14) d'évacuation du second dispositif de détente (F03) pour la solution lavante purifiée.

11. Installation selon la revendication 10, **caractérisée en ce que** pour le réchauffement par étape de la solution lavante, deux échangeurs de chaleur (W02, W03) montés en série sont disposés avant le premier dispositif de détente (F01).

12. Installation selon l'une quelconque des revendications 10 ou 11, **caractérisée en ce que** les dispositifs de détente (F01, F03) sont reliés à des conduites (06, 13) pour l'évacuation d'un courant de gaz (CO₂ et composés de souffre).

13. Installation selon l'une quelconque des revendications 10 à 12, **caractérisée en ce que** la conduite de courant de gaz (06) du premier dispositif de détente (F01) est reliée à un séparateur (F02), deux échangeurs de chaleur (W06, W07) montés en série pour le refroidissement du courant de gaz étant intégrés entre le dispositif de détente (F01) et le séparateur (F02), le séparateur (F02) étant relié à une conduite (08) pour l'évacuation du courant de gaz et à une conduite (07) pour le transport de l'eau de condensation séparée dans le second dispositif de détente (F03).

14. Installation selon l'une quelconque des revendications 10 à 13, **caractérisée en ce qu'**au moins l'un des échangeurs de chaleur (W02) placé en amont est intégré dans la conduite (12) d'embranchement du premier dispositif de détente (F01) pour la solution lavante, la solution lavante traversant ledit échangeur sous forme d'agent caloporteur.

15. Installation selon l'une quelconque des revendications 10 à 14, **caractérisée en ce qu'**au moins la conduite de courant de gaz (08) embranchée sur le séparateur (F02) placé à la suite est raccordée à un dispositif de désulfuration.
